# FASCICULE DE BREVET EUROPEEN

(11) **EP 2 079 837 B1**
(45) Date de publication et mention de la délivrance du brevet: **04.06.2014**
(21) Numéro de dépôt: 07820948.3
(22) Date de dépôt: 04.10.2007
(51) Int. Cl.: C12N 15/01, C12N 1/20, C12P 7/66, A23L 1/03, A23L 1/302, A23C 9/123, C12R 1/46

(54) **PROCEDE D'OBTENTION DE VARIANTS DE BACTERIES LACTIQUES UTILES POUR PRODUIRE LA VITAMINE K2 ET APPLICATIONS A LA PREPARATION DE PRODUITS ALIMENTAIRES**
VERFAHREN ZUR GEWINNUNG VON MILCHSÄUREBAKTERIENVARIANTEN GEEIGNET FÜR DIE PRODUKTION VON VITAMIN K2 UND VERWENDUNG ZUR HERSTELLUNG VON LEBENSMITTELN
METHOD FOR OBTAINING VARIANTS OF LACTIC BACTERIA USEFUL IN THE PRODUCTION OF VITAMIN K2 AND APPLICATIONS FOR THE PREPARATION OF FOOD PRODUCTS

(30) Priorité: 04.10.2006 FR 0608690
(43) Date de publication de la demande: 22.07.2009
(73) Titulaire: Compagnie Gervais Danone, 75009 Paris (FR)
(72) Inventeur: GARAULT, Peggy, F-91310 Montlhery (FR); QUERE, Gaëlle, F-91140 Villebon Sur Yvette (FR); BEAL, Chloé, F-75014 Paris (FR); BOMCHIL, Natalia, F-69008 Lyon (FR); FAURIE, Jean-Michel, F-78350 Jouy En Josas (FR); GOBERT, Guillaume, F-91120 Palaiseau (FR); LIPOWSKI, Gérard, F-92130 Issy Les Moulineaux (FR)
(74) Mandataire: Regimbeau
(86) Numéro de dépôt international: PCT/EP2007/060572
(87) Numéro de publication internationale: WO 2008/040793

(56) Documents cités:
- JP-A- 62 201 592
- JP-A- 63 091 091
- JP-A- 63 198 993
- JP-A- 2000 287 676
- MORISHITA T: "PRODUCTION OF MENAQUINONES BY LACTIC ACID BACTERIA" JOURNAL OF DAIRY SCIENCE, AMERICAN DAIRY SCIENCE ASSOCIATION, SAVOY, IL, US, vol. 82, no. 9, 1999, pages 1897-1903, XP000983779 ISSN: 0022-0302 cité dans la demande
- VIDO KARIN ET AL: "Roles of thioredoxin reductase during the aerobic life of Lactococcus lactis" JOURNAL OF BACTERIOLOGY, vol. 187, no. 2, janvier 2005 (2005-01), pages 601-610, XP002430548 ISSN: 0021-9193 cité dans la demande

## Description

La présente invention se rapporte au domaine des produits alimentaires riches en nutriments, vitamines et/ou oligo-éléments afin d'améliorer la teneur et l'équilibre qualitatif et quantitatif des apports nutritionnels chez l'homme.

L'invention s'intéresse plus particulièrement aux moyens d'enrichir les aliments en vitamine K.

Plus précisément, la présente invention concerne l'obtention de variants de souches de bactéries lactiques qui produisent, dans des conditions de fermentation standard, au moins environ 1,2 fois plus de vitamine K2 que les souches de bactéries lactiques de départ cultivées dans les mêmes conditions.

L'invention concerne également un procédé de préparation de produits alimentaires, notamment de produits fermentés et/ou de produits laitiers frais, enrichis en vitamine K2, ainsi que les produits alimentaires ainsi obtenus.

La vitamine K est une vitamine liposoluble qui se présente sous deux formes naturelles : la vitamine K1 (ou phylloquinone) et la vitamine K2 (ou ménaquinone)

La vitamine K1 est synthétisée par les végétaux. On la trouve principalement dans les légumes verts (légumes-feuilles) et l'huile de soja. La vitamine K1 intervient plus directement dans le processus de coagulation du sang.

La vitamine K2, quant à elle, est produite par les bactéries de la flore intestinale. Elle apparaît également en faibles quantités dans certains aliments à la suite d'un processus de fermentation (fromage, produits asiatiques typiques tels que le miso et le natto japonais, à base de soja fermenté, etc.). De nombreuses bactéries sont capables de synthétiser la vitamine K2. Ainsi, outre les bactéries de la flore intestinale et, notamment, les espèces *Escherichia coli, Bacillus subtilis* et *Bacteroides spp.,* on peut citer certaines espèces ou sous-espèces de bactéries lactiques telles que *Lactococcus lactis* spp. *lactis, Lactococcus lactis* spp. *cremoris, Leuconostoc lactis, Leuconostoc mesenteroides* et *Propionibacterium* sp. La quantité de vitamine K2 synthétisée par ces bactéries varie généralement d'environ 29 à 90 µg/l de lait fermenté (Morishita et al., 1999). Il est important de souligner que les mesures de production de vitamine K2 sont le plus souvent réalisées à partir de lyophilisats de culots cellulaires et les résultats de ces mesures révèle une grande hétérogénéité des niveaux de production en fonction des souches testées, pouvant varier du simple à plus du triple (Morishita et al., 1999 ; Parker et al., 2003). En termes d'activité biologique, la vitamine K2 est surtout connue pour son action sur la calcification des tissus mous.

La vitamine K a initialement été décrite pour son rôle essentiel dans le processus de coagulation du sang. Ainsi, de fortes carences en vitamine K entraînent des hémorragies, avec allongement anormal du temps de coagulation, et des ecchymoses. On a longtemps considéré que les carences fortes en vitamine K étaient plutôt rares chez l'adulte, les besoins pouvant être en principe couverts de manière satisfaisante par une alimentation variée et équilibrée et grâce à la production endogène de la vitamine par les bactéries coliques. A cet égard, les personnes à risque sont typiquement :
- les nouveaux-nés, dont les intestins ne possèdent pas à la naissance les bactéries productrices de vitamine K ;
- les personnes dont les fonctions hépatiques, biliaires ou intestinales sont perturbées (maladies hépatiques, mucoviscidose, colites, dysenteries...) ; et
- celles qui prennent des antibiotiques au long cours.

Plus récemment, on a découvert que l'impact de la vitamine K sur la santé humaine ne se limitait pas à son rôle dans les mécanismes de coagulation sanguine. En effet, depuis les années 80, la vitamine K est également reconnue pour son rôle dans le métabolisme osseux (Hart et al., 1984 ; Hart et al., 1985).

Cette vitamine joue le rôle de cofacteur dans une réaction enzymatique conditionnant l'activité de l'ostéocalcine dans le cadre de la régulation de la formation osseuse (Hauschka PV et al., 1989; Ducy P et al., 1996). Son rôle consiste plus précisément à conditionner la carboxylation de l'ostéocalcine, protéine-clé qui régule le processus de formation osseuse. En cas de déficience en vitamine K, cette réaction n'a pas lieu, entraînant l'augmentation du ratio sanguin ostéocalcine décarboxylée sur ostéocalcine carboxylée (Väänänen *et al.,* 1999).

L'évolution démographique des pays occidentaux s'est traduite par un vieillissement progressif des populations, associé en corollaire à une augmentation de la prévalence des pathologies dégénératives, notamment de l'ostéoporose. A ce titre, l'ostéoporose est désormais reconnue comme un problème majeur de santé publique.

Les estimations démographiques établies dans les années 90 ont tiré la sonnette d'alarme en prévoyant une augmentation considérable de l'incidence de cette pathologie dans les 50 ans à venir, notamment chez les seniors. S'est donc rapidement imposée la nécessité et l'urgence d'entreprendre des actions afin de prévenir cette pathologie, jusque-là rarement dépistée et prise en charge tardivement.

Il est désormais reconnu que la prévention de l'ostéoporose doit commencer dès l'enfance, au travers d'une croissance osseuse optimale, et se poursuivre toute la vie par un maintien de la masse osseuse. On sait que les facteurs nutritionnels jouent un rôle important dans le développement et le maintien du patrimoine osseux. Jusqu'à présent, les stratégies nutritionnelles envisagées ou proposées pour prévenir l'ostéoporose reposent essentiellement sur deux facteurs clés qui sont le calcium et la vitamine D. Pourtant, on sait aujourd'hui que d'autres facteurs nutritionnels pourraient présenter un intérêt notable.

De par son rôle majeur dans la formation osseuse, la vitamine K apparaît de plus en plus dans la littérature comme une piste prometteuse pour préserver la santé osseuse de l'homme tout au long de sa vie.

Les apports nutritionnels recommandés en vitamine K chez l'homme (1,5 µg/j/kg de poids) ont été établis en ne prenant en considération que son rôle dans les phénomènes de coagulation. Or, des études récentes suggèrent que ces apports nutritionnels recommandés sont finalement sous-estimés si l'on prend également en compte l'activité de la vitamine K dans le métabolisme osseux (Ronden et al., 1998).

Si les besoins en vitamine K sont encore mal connus, il n'en demeure pas moins que de faibles apports sont associés à une faible masse osseuse et à un risque accru de fractures chez l'adulte (Hart et al., 1985 ; Knapen et al., 1989 ; Szulc et al., 1993 ; Booth et al, 2000). De plus, des études d'intervention chez les femmes ménopausées ont montré que la vitamine K pouvait permettre de diminuer les pertes osseuses pour cette cible (Shiraki et al., 2000 ; Braam et al., 2003). Enfin, des études chez l'animal suggèrent qu'elle pourrait jouer un rôle favorable au cours du pic de masse osseuse et ce, d'autant mieux en cas d'association synergique avec la vitamine D. Cependant, les études reliant clairement la vitamine K et la croissance osseuse n'ont pour l'heure été menées que chez l'animal.

De plus, des études récentes ont permis d'apporter des arguments supplémentaires en faveur de l'impact de la vitamine K sur le métabolisme osseux et, en particulier, sur la constitution et la préservation de la masse osseuse (Booth et al., 2000; Shiraki et al., 2000; Braam et al., 2003; Hirano et Ishi, 2002).

Contrairement à l'adulte, peu de données sont encore disponibles en ce qui concerne les effets bénéfiques de la vitamine K sur le métabolisme osseux de l'enfant. On sait seulement qu'il est essentiel d'optimiser la masse osseuse pendant la période de croissance, afin de constituer un réservoir osseux maximal et de protéger l'adulte contre le risque d'ostéoporose à venir.

En tout état de cause, il ressort de l'ensemble des données disponibles à ce jour que l'amélioration de la teneur en vitamine K des produits alimentaires est une piste particulièrement intéressante et prometteuse pour permettre à l'individu de construire et maintenir une bonne constitution osseuse.

Dans ce contexte, il existe déjà des produits industriels sur le marché de l'alimentaire contenant une quantité notable de vitamine K. On peut notamment citer certains produits laitiers renfermant des bactéries lactiques, tels que les « Petits Gervais aux Fruits » commercialisés en France par la Demanderesse. On notera néanmoins que, d'une part, la teneur de ces produits en vitamine K dépend généralement du type de ferments utilisés et, d'autre part, les souches de *Lactococcus lactis* classiquement utilisées dans les produits laitiers ne produisent pas une quantité suffisante de vitamine K pour véritablement combler les besoins de la population, voire pour aider à pallier d'éventuelles carences en vitamine K.

Il existe donc un besoin dans la technique actuelle pour des produits alimentaires, notamment des produits fermentés et/ou des produits laitiers frais, qui renferment de la vitamine K en quantités suffisantes pour contribuer à satisfaire les besoins et, si nécessaire, à combler les carences, tant chez l'enfant et l'adolescent, que chez l'adulte et la personne âgée.

Dans ce qui suit, les termes « vitamine K2 » et « vitamine K » sont utilisés indifféremment pour désigner la vitamine K2.

La présente invention vise donc à répondre à ce besoin en proposant de préparer des produits alimentaires, notamment des produits fermentés et/ou des produits laitiers frais, en utilisant de nouveaux variants de souches de bactéries lactiques, qui produisent des quantités de vitamine K significativement supérieures à celles produites par les souches desquelles ils dérivent.

En outre, au cours de leurs travaux, les Inventeurs ont mis au point des conditions de mise en oeuvre des bactéries lactiques qui favorisent de manière tout à fait sensible la production de vitamine K par rapport aux conditions de production classiques. Ainsi, aux fins de la préparation de produits alimentaires, tels que des produits fermentés et/ou des produits laitiers frais, enrichis en vitamine K2, on pourra avantageusement utiliser les variants « surproducteurs » de vitamine K qui font l'objet de la présente invention dans les conditions de mise en oeuvre identifiées par les Inventeurs comme étant particulièrement favorables à la production de vitamine K et faisant l'objet de la demande de brevet français n°06 / 08690 du 4 octobre 2006.

L'équipe de Morishita et al. (J. Dairy Science, 1999) a décrit des souches de bactéries lactiques produisant de la vitamine K2 (« menaquinone »). Les auteurs ne proposent pas d'utiliser un milieu de culture contenant de la bacitracine ou un agent oxydant. Les bactéries décrites ne sont donc pas résistantes à ces agents.

La demande JP 2000-287676 décrit un procédé d'obtention d'un variant naturel d'une souche de bactérie de l'espèce *Bacillus Nato* produisant de la vitamine K2. Ce procédé n'utilise pas de milieu sélectif contenant de la bacitracine ou un agent oxydant.

Le terme « varian » couvre ici :
- les variants naturels, c'est-à-dire obtenus spontanément à partir d'une souche de bactérie lactique de référence sous l'effet d'une pression de sélection ; les variants naturels ne subissent donc aucune manipulation génétique, mais sont obtenus principalement par mutation et sélection à partir de la souche de référence ; et
- les mutants comprenant une ou plusieurs mutations dans leur génome, qui ont été induites par génie génétique, c'est-à-dire par des techniques de mutagénèse dirigée, notamment par transformation génétique à l'aide de vecteurs, appliquées à la souche de référence.

On notera que, dans certains pays (notamment en Europe), des précautions doivent être prises par les industriels de l'alimentaire lorsqu'ils développent des produits destinés à l'alimentation humaine et/ou animale, dans lesquels sont incorporés des microorganismes, plus particulièrement des microorganismes vivants. En effet, les organismes (ici, microorganismes) génétiquement modifiés (OGM ou mutants) peuvent susciter un sentiment de crainte et d'appréhension chez les consommateurs. Cette image négative dont souffrent les OGM dans certains pays est telle que le public a tendance à « boycotter » les aliments contenant des OGM. Aussi, dans un contexte où les consommateurs exigent toujours plus de transparence aux niveaux du contenu des produits alimentaires qui leur sont proposés et de l'origine des ingrédients que ces produits contiennent, les industriels peuvent être amenés à proposer des produits quasi-exclusivement, voire exclusivement, sans OGM. Dans le contexte de la présente invention, il peut donc être avantageux que les produits alimentaires issus de l'industrie et contenant des microorganismes soient préparés en utilisant exclusivement des souches naturelles ou des variants naturels de souches naturelles.

Selon un premier aspect, la présente invention concerne un procédé de sélection d'un variant naturel d'une souche de bactérie lactique produisant, dans des conditions de fermentation standard, une quantité de vitamine K2 supérieure, d'un facteur au moins égal à environ 1,2, à celle produite par ladite souche de bactérie lactique cultivée dans les mêmes conditions, ledit procédé comprenant au moins :
a) la culture de ladite souche de bactérie lactique dans des conditions de fermentation standard, sur un milieu de sélection choisi parmi les milieux de culture contenant de la bacitracine ou un agent oxydant tel que le péroxyde induisant une modification de l'état d'oxydo-réduction cellulaire; et
b) la sélection dudit variant s'il produit au moins environ 1,2 fois plus de vitamine K2 que ladite souche de bactérie lactique cultivée dans les mêmes conditions.

Les « variants » sont, au sens de l'invention, des souches de bactéries lactiques capables de produire plus de vitamine K2 que les souches dont elles sont issues. Plus précisément, les variants selon la présente invention sont capables de produire au moins environ 1,2 fois plus de vitamine K2 que les souches de départ. De préférence, la quantité de vitamine K2 produite par un variant conforme à la présente invention est supérieure, d'un facteur au moins égal à environ 1,5, à celle obtenue en cultivant la souche de bactérie lactique de départ dans les mêmes conditions de fermentation standard. Ce facteur est de manière encore préférée au moins égal à environ 1,7, plus préférentiellement au moins égal à environ 1,8, plus préférentiellement encore au moins égal à environ 1,9. Des valeurs de ce facteur encore davantage préférées sont d'au moins environ 2 ; 2,2 ; 2,4 ; 2,5 ; 2,7 ; 2,8 ; 2,9 ; 3.

Les souches de bactéries lactiques « de référence » ou « de départ » sont les souches à partir desquelles les variants selon l'invention sont obtenus. Ces souches peuvent être naturelles ou bien être elles-mêmes des variants, c'est-à-dire des variants naturels ou des mutants.

Dans le cadre de la présente invention, les « conditions de laboratoire » sont des conditions de fermentation tout à fait standard et bien connues de l'homme du métier. Ainsi, les expressions « conditions de laboratoire » et « conditions de fermentation standard » sont ici parfaitement synonymes. Les « conditions de laboratoire » préférées au sens de la présente invention sont les suivantes : une préculture de la souche est réalisée sur milieu commercial M17 (Difco™ M17) ou sur un milieu équivalent. Pour la culture qui suit, l'ensemencement est réalisé à 1% à l'aide de la préculture. La température d'incubation est de 30°C environ. On retiendra que les conditions de laboratoire peuvent être modifiées en cas de besoin par l'homme du métier, sur la base de ses connaissances générales et, éventuellement, après des expériences de mises au point de routine. Le milieu de culture est un milieu approprié pour cultiver les souches de bactéries lactiques, notamment les souches de *Lactococcus spp.*

Selon un mode de réalisation préféré, la souche de bactérie lactique est choisie parmi les genres *Lactococcus, Leuconostoc, Enterococcus* et *Propionibacterium.* Elle est plus particulièrement choisie parmi les espèces *Lactococcus lactis, Leuconostoc lactis, Leuconostoc pseudomesenteroides, Leuconostoc mesenteroides, Leuconostoc dextranicum, Enterococcus faecium, Propionibacterium sp.*

Comme cela est développé dans ce qui suit, dans le procédé selon l'invention, on met en oeuvre un milieu de sélection choisi parmi des milieux de culture contenant de la bacitracine ou un agent oxydant tel que le péroxyde.

Le milieu de sélection ainsi mis en oeuvre permet d'induire une modification de l'état d'oxydo-réduction de la cellule. Avantageusement, cette modification est corrélée à la modification, chez ledit variant et par comparaison avec la souche de bactérie lactique dont il est issu, de l'expression d'au moins un gène choisi parmi les gènes n° 1 à 27 listés dans le tableau I suivant:

**Tableau I**

| **N° du gène** | **Nom du gène** | **Fonction de la protéine correspondante** | **N° d'accès NCBI** |
|---|---|---|---|
| 1 | cys D | O-acétylhomosérine sulfhydrylase | IImg_0091 |
| 2 | gpo | Gpo protéine (glutathione peroxydase) | IImg_1088 |
| 3 | metE | 5-méthyltetrahydropteroyltriglutamate-- homocystéine méthyltransférase | IImg_1225 |
| 4 | | NADH déshydrogénase putative | IImg_0195 |
| 5 | metF | Protéine MetF | IImg_1226 |
| | | *MetF protein* | |
| | | *Méthylène tétrahydrofolate réductase* | |
| 6 | trxH | Thiorédoxine type H | IImg_0406 |
| 7 | fur | Protéine de régulation de l'assimilation ferrique | IImg_1023 |
| 8 | qor | quinone oxydoréductase | IImg_1850 |
| 9 | frdc | sous-unité flavoprotéine fumarate réductase | IImg_1441 |
| 10 | adhE | alcool-acétaldéhyde déshydrogénase | IImg_2432 |
| 11 | purC | phosphoribosylaminoimidazole-succinocarboxamide synthase | IImg_0973 |
| 12 | cydB | cytochrome d ubiquinol oxydase, sous-unité II | IImg_1863 |
| 13 | purE | Sous-unité catalytique phosphoribosylaminoimidazole carboxylase | IImg_0999 |
| 14 | purQ | phosphoribosylformylglycinamidine synthase I | IImg_0975 |
| 15 | trxA | Thiorédoxine | IImg_0779 |
| 16 | noxB | NADH déshydrogénase | IImg_1734 |
| 17 | cpo | non-hème chlorure péroxidase | IImg_1737 |
| 18 | metK | Protéine MetK S-adénosylméthionine synthase | IImg_2160 |
| 19 | trxB1 | Protéine TrxB1 Thiorédoxine réductase | IImg_1588 |
| 20 | cysK | O-acétylsérine sulfhydrylase | IImg_1775 |
| 21 | metC | cystathionine beta-lyase | IImg_1776 |
| 22 | metS | Protéine MetS Méthionyl-tRNA synthétase | IImg_1764 |
| 23 | feoB | Homologue de la protéine B de transport de fer ferreux. | IImg_0199 |
| 24 | citB | aconitate hydratase | IImg_0636 |
| 25 | icd | isocitrate déshydrogénase | IImg_0637 |
| 26 | fhuD | Protéine affine du substrat du système de transport ABC du ferrichrome. | IImg_0349 |
| 27 | Idh | L-lactate déshydrogénase | IImg_1120 |

De préférence, l'expression des gènes n°1 à 27 est modifiée chez ledit variant par rapport à la souche de la bactérie lactique.

Par « modification de l'expression d'un gène », on veut ici dire que l'expression du gène considéré est quantitativement modifiée par rapport à celle observée chez la souche de bactérie lactique de départ :
- soit l'expression est augmentée, et ce sera de préférence le cas pour au moins un gène choisi parmi les gènes n°1 à 15 ;
- soit l'expression est réduite, et ce sera de préférence le cas pour au moins un gène choisi parmi les gènes n°16 à 27.

De préférence, le variant est sélectionné à l'étape b) s'il produit, dans des conditions de fermentation standard :
- au moins 1,5 fois plus environ, de préférence au moins 2 fois plus environ, de préférence encore au moins 3 fois plus environ, de vitamine K2 que le ferment CHN-12; et/ou
- au moins 1,5 fois plus environ, de préférence au moins 2 fois plus environ, de préférence encore au moins 3 fois plus environ et, avantageusement jusqu'à au moins 10 fois plus environ, de vitamine K2 que la souche naturelle modèle MG1363.

Avantageusement, le variant est sélectionné à l'étape b) s'il produit, dans des conditions de fermentation standard, au moins 5,5 µg environ de vitamine K2 pour 100g de lait fermenté.

Si la quantité de vitamine K2 produite par les variants est d'au moins 5,5 µg environ pour 100g de lait fermenté dans des conditions de fermentation standard, on pourra parler de variant « surproducteur » de vitamine K2. En particulier, un variant au sens de la présente invention produit au moins 5,7 µg environ, de préférence encore au moins 5,9 µg environ, de manière encore préférée au moins 6,1 µg environ, et, mieux, au moins 6,3 µg environ de vitamine K2 pour 100g de lait fermenté dans des conditions de fermentation standard. Plus préférentiellement, un variant selon la présente invention produit au moins 6,5 µg environ, de préférence au moins 7 µg environ, de préférence encore au moins 7,5 µg environ, de manière plus préférée au moins 8 µg environ, de manière plus préférée encore au moins 8,5 µg environ, de manière encore davantage préférée au moins 9 µg environ, mieux, au moins 9,5 µg environ et, encore mieux, au moins 10 µg environ de vitamine K2 pour 100g de lait fermenté dans des conditions de fermentation standard.

Dans un deuxième aspect, la présente invention concerne un variant naturel d'une souche de bactérie lactique résistant à la bacitracine ou à un agent oxydant tel que le péroxyde, susceptible d'être obtenu par un procédé tel que décrit ci-dessus, ainsi que les cultures et fractions de cultures biologiquement pures dudit variant, ledit variant produisant, dans des conditions de fermentation standard, une quantité de vitamine K2 supérieure, d'un facteur au moins égal à environ 1,2, à celle produite par la souche de bactérie lactique de départ cultivée dans les mêmes conditions.

En particulier, un variant conforme à la présente invention produit, dans des conditions de laboratoire (ou conditions de fermentation standard) :
- au moins environ 1,5 fois plus, de préférence au moins environ 2 fois plus, de préférence encore au moins environ 3 fois plus, de vitamine K2 que le ferment CHN-12 commercialisé par CHR. Hansen A/S (Horsholm, DK) ; et/ou
- au moins environ 1,5 fois plus, de préférence au moins environ 2 fois plus, de préférence encore au moins environ 3 fois plus et, avantageusement jusqu'à au moins environ 10 fois plus, de vitamine K2 que la souche naturelle modèle *Lactococcus lactis ssp. cremoris* MG1363 déposée au CBS (Baarn, NL) sous le numéro CBS 364.89. Cette souche modèle de *Lactococcus lactis ssp. cremoris* est parfaitement connue de l'homme du métier. Elle a été décrite pour la première fois par Gasson en 1983 (Gasson M., 1983).

En accord avec la définition de « variant naturel » donnée plus haut, un variant selon la présente invention est un variant naturel obtenu par pression de sélection sur un milieu de culture approprié.

Plusieurs exemples de tels variants naturels obtenus par pression de sélection sont fournis dans la présente demande. Brièvement (pour plus de détails, voir la partie « Exemples » ci-dessous), des exemples préférés de variants naturels et méthodes pour les obtenir sont les suivants.

Selon un premier mode de réalisation, un variant naturel conforme à l'invention est obtenu par pression de sélection sur un milieu de culture contenant de la bacitracine. En fonction des souches de départ, la concentration de bacitracine dans le milieu peut être, par exemple, d'au moins 0,4 mg/l environ, de préférence d'au moins 1 mg/l environ, de préférence encore d'au moins 2 mg/l environ, de manière encore préférée d'au moins 3 mg/l environ et de manière préférée entre toutes d'au moins 4 mg/l environ. Cependant, il est clair pour l'homme du métier que la concentration de bacitracine à utiliser pour obtenir des variants naturels conformes à l'invention, sera déterminée en fonction du niveau de résistance à la bacitracine de la souche de bactérie lactique utilisée au départ. Si nécessaire, l'homme du métier travaillera à plusieurs concentrations différentes qu'il choisira en fonction des propriétés de la souche de départ. De manière avantageuse, l'homme du métier pourra travailler avec des gammes de concentrations de bacitracine.

Un variant naturel particulièrement intéressant présente essentiellement les mêmes propriétés biologiques que le variant naturel I-3557, déposé à la Collection Nationale de Culture des Microorganismes (CNCM, Institut Pasteur, 25 rue du Docteur Roux, 75724 Paris cedex 15, France) le 20/01/2006.

Par l'expression « un variant A présente essentiellement les mêmes propriétés biologiques que le variant naturel I-3557 », on entend ici que le variant A est un variant naturel obtenu par pression de sélection sur un milieu comprenant de la bacitracine. Toutefois, la concentration de bacitracine mise en oeuvre dans le milieu pour obtenir le variant A n'est pas déterminante pour satisfaire à la présente définition. La condition déterminante, en revanche, est que le variant A soit capable de produire, dans les conditions de laboratoire, approximativement autant, de préférence au moins autant, de vitamine K2 que le variant I-3557. Un variant naturel au sens de l'invention est de préférence le variant naturel I-3557.

Dans un deuxième mode de réalisation, un variant naturel selon la présente invention est obtenu par pression de sélection sur un milieu de culture contenant au moins un agent oxydant. Par exemple, l'agent oxydant peut être choisi parmi le péroxyde, les ions hyperchloriques, les ions ferreux, la ménadione, le paraquat, l'oxygène ou n'importe quel autre composé oxydant approprié. De préférence, l'agent oxydant est du péroxyde. Comme pour la bacitracine, la concentration de péroxyde dans le milieu est déterminée en fonction de la souche de bactérie lactique de départ. Par exemple, on pourra tester une ou plusieurs concentrations de péroxyde dans les gammes suivantes : au moins 20, 25, 27, 28,5 mg/l environ. Encore une fois, l'homme du métier déterminera une ou plusieurs concentrations, voire une ou plusieurs gammes de concentrations, appropriées de péroxyde qu'il testera expérimentalement comme il en a l'habitude.

Avantageusement, un variant naturel conforme à l'invention présente essentiellement les mêmes propriétés biologiques que le variant naturel I-3558 (déposé à la CNCM le 20/01/2006). La définition de l'expression « un variant A présente essentiellement les mêmes propriétés biologiques que le variant naturel I-3557 » donnée plus haut, s'applique ici *mutatis mutandis* (péroxyde au lieu de bacitracine ; variant I-3558 au lieu du variant I-3557). De préférence, un tel variant est le variant naturel I-3558.

Un troisième aspect de la présente invention vise des utilisations de conditions de sélection particulières pour sélectionner des variants naturels de souches de bactéries lactiques qui, conformément à la description qui précède, produisent, dans des conditions de fermentation standard, au moins 1,2 fois plus environ de vitamine K2 que les souches de départ cultivées dans les mêmes conditions.

Ces utilisations sont notamment :
- l'utilisation de la résistance à la bacitracine ; et/ou
- l'utilisation de la résistance à un agent oxydant, tel que le péroxyde.

Comme indiqué précédemment; lesdits variants naturels sont avantageusement capables de produire au moins 5,5 µg environ de vitamine K2 pour 100g de lait fermenté dans des conditions de fermentation standard.

Un quatrième aspect de la présente invention a trait à un ferment lactique qui comprend au moins un variant tel que décrit supra.

Selon un cinquième aspect, la présente invention concerne un procédé de production d'un produit alimentaire enrichi en vitamine K2, comprenant au moins :
a) la mise en oeuvre d'au moins un variant et/ou au moins un ferment tel(s) que décrit(s) plus haut, dans une préparation intermédiaire dudit produit ; et
b) l'obtention dudit produit enrichi en vitamine K2.

Alternativement, un procédé pour augmenter la teneur en vitamine K2 d'un produit alimentaire comprend au moins :
a) la mise en oeuvre d'au moins un variant et/ou au moins un ferment tel(s) que décrit(s) plus haut, dans une préparation intermédiaire dudit produit ; et
b) l'obtention dudit produit enrichi en vitamine K2.

Les variants et/ou le ferment lactique peuvent notamment être mis en oeuvre en utilisant des concentrats de bactéries précultivées sur place (sur le site de production des produits alimentaires), ou en utilisant des bactéries précultivées par un fournisseur de ferments, puis conditionnées et expédiées vers le(s) site(s) de production des produits alimentaires. Les fournisseurs peuvent conditionner les bactéries à l'état frais ou congelé ; alternativement, les bactéries peuvent être séchées ou lyophilisées. Les bactéries sont, dans tous les cas, ajoutées à la masse laitière de manière tout à fait classique (comme n'importe quel autre ferment lactique connu).

Un sixième aspect de la présente invention vise un produit alimentaire enrichi en vitamine K2 comprenant au moins un variant selon l'invention et/ou au moins un ferment selon l'invention, susceptible d'être obtenu par un procédé tel que divulgué ci-dessus. L'invention s'intéresse aux produits alimentaires pour l'homme et/ou l'animal, avec une préférence pour les produits destinés à l'alimentation humaine. Avantageusement, un tel produit alimentaire enrichi en vitamine K2 renforce la solidité des os de la personne qui le consomme. Cette personne est, de manière préférée, un enfant.

De préférence, un produit alimentaire au sens de l'invention est choisi parmi les produits fermentés, les produits laitiers frais fermentés ou non, les produits à base de jus d'origine végétale (fruits, légumes, céréales, soja, etc.) fermentés ou non, et leurs combinaisons. De manière plus particulièrement préférée, un produit alimentaire au sens de l'invention est un produit fermenté et/ou un produit laitier frais.

Dans le contexte de l'invention, les « produits laitiers frais » désignent plus particulièrement des produits laitiers frais et fermentés, prêts à la consommation humaine, c'est-à-dire des aliments laitiers frais et fermentés. Dans la présente demande, sont plus particulièrement visés les laits fermentés et yoghourts. Lesdits aliments laitiers frais et fermentés peuvent alternativement être des fromages blancs ou des petits-suisses.

On donne aux termes « laits fermentés » et « yoghourts » leurs significations usuelles dans le domaine de l'industrie laitière, c'est-à-dire des produits qui sont destinés à la consommation humaine et qui sont issus de la fermentation lactique acidifiante d'un substrat laitier. Ces produits peuvent contenir des ingrédients secondaires tels que fruits, végétaux, sucre, etc. On peut, par exemple, se reporter au Décret français n° 88-1203 du 30 décembre 1988 relatif aux laits fermentés et au yaourt ou yoghourt, publié au Journal Officiel de la République Française du 31 décembre 1988.

On peut également se reporter au « Codex Alimentarius » (préparé par la Commission du Codex Alimentarius sous l'égide de la FAO et de l'OMS, et publié par la Division Information de la FAO, disponible en ligne sur http://www.codexalimentarius.net; cf. plus particulièrement le volume 12 du Codex Alimentarius « Normes Codex pour le lait et les produits laitiers », et la norme « CODEX STAN A -1 1(a)-1975 »).

L'expression « lait fermenté » est ainsi réservée dans la présente demande au produit laitier préparé avec un substrat laitier qui a subi un traitement au moins équivalent à la pasteurisation, ensemencé avec des microorganismes appartenant à l'espèce ou aux espèces caractéristique(s) de chaque produit. Un « lait fermenté » n'a subi aucun traitement permettant de soustraire un élément constitutif du substrat laitier mis en oeuvre et n'a notamment pas subi un égouttage du coagulum. La coagulation des « laits fermentés » ne doit pas être obtenue par d'autres moyens que ceux qui résultent de l'activité des microorganismes utilisés.

Le terme « yoghourt » est quant à lui réservé au lait fermenté obtenu, selon les usages locaux et constants, par le développement des bactéries lactiques thermophiles spécifiques dites *Lactobacillus bulgaricus* et *Streptococcus thermophilus,* qui doivent se retrouver vivantes dans le produit fini, à raison d'au moins 10 millions de bactéries par gramme rapportées à la partie lactée.

Dans certains pays, la réglementation autorise l'ajout d'autres bactéries lactiques dans la production de yoghourt, et notamment l'utilisation additionnelle de souches de *Bifidobacterium* et/ou *Lactobacillus acidophiles* et/ou *Lactobacillus casei.* Ces souches lactiques additionnelles sont destinées à conférer au produit fini diverses propriétés, telles que celle de favoriser l'équilibre de la flore intestinale ou de moduler le système immunitaire.

Dans la pratique, l'expression « lait fermenté » est donc généralement utilisée pour désigner les laits fermentés autres que les yoghourts. Elle peut d'ailleurs prendre, selon les pays, des noms aussi divers que, par exemple, « Kefir », « Kumiss », « Lassi », « Dahi », « Leben », « Filmjôlk », « Villi », « Acidophilus milk ».

S'agissant des laits fermentés, la quantité d'acide lactique libre contenue dans le substrat laitier fermenté ne doit pas être inférieure à 0,6 g pour 100 g lors de la vente au consommateur, et la teneur en matière protéique apportée à la partie lactée ne doit pas être inférieure à celle d'un lait normal.

Enfin, la dénomination « fromage blanc » ou « petit-suisse » est, dans la présente demande, réservée à un fromage non affiné, non salé, qui a subi une fermentation par des bactéries lactiques uniquement (et aucune autre fermentation que la fermentation lactique). La teneur en matière sèche des fromages blancs peut être abaissée jusqu'à 15 g ou 10 g pour 100 g de fromage blanc, selon que leur teneur en matières grasses est supérieure à 20 g, ou au plus égale à 20 g pour 100 g de fromage blanc, après complète dessication. La teneur en matière sèche d'un fromage blanc est comprise entre 13 et 20 %. La teneur en matière sèche d'un petit-suisse quant à elle n'est pas inférieure à 23 g pour 100 g de petit-suisse. Elle est généralement comprise entre 25 et 30 %. Les fromages blancs et petits-suisses sont généralement regroupés sous la dénomination « fromages frais », utilisée de manière classique dans le domaine technique de la présente invention.

Dans un septième aspect, la présente invention concerne l'utilisation d'au moins un variant et/ou au moins un ferment comme décrit(s) plus haut, pour préparer un produit alimentaire enrichi en vitamine K2.

Avantageusement, un tel produit alimentaire enrichi en vitamine K2 renforce la solidité des os de la personne qui le consomme. Cette personne est, de manière préférée, un enfant.

Il est clair que la présente invention ne se limite pas à la seule description ci-dessus. D'autres modes de réalisation et avantages de l'invention pourront ressortir à la lecture des exemples ci-dessous, fournis à titre purement illustratif.

### EXEMPLES

A titre de remarques préliminaires, il est à noter que les protocoles d'obtention de variants naturels décrits ci-dessous sont applicables à n'importe quel type de souche de bactérie lactique de départ. En fonction des souches de départ que l'homme du métier utilisera, il pourra éventuellement être amené, pour des raisons essentiellement pratiques, à modifier certaines des conditions expérimentales mises au point par les Inventeurs. En tout état de cause, les modifications que l'homme du métier sera susceptible d'apporter aux procédures ci-dessous seront mineures et nécessiteront uniquement de simples manipulations de routine n'impliquant aucune activité inventive.

### I- Obtention et utilisation de variants naturels résistants à la bacitracine

Bien qu'une exposition à des agents tels que la bacitracine ou le péroxyde soit connu pour permettre de sélectionner des souches bactériennes qui présentent une résistance accrue à ces agents, il n'a jamais été établi de lien dans la littérature entre la résistance à la bacitracine ou au péroxyde et les niveaux de production de vitamine K2 par les bactéries.

Dans le cadre de leurs travaux, les Inventeurs ont découvert de manière tout à fait inattendue que les bactéries étaient capables de développer un mécanisme original de résistance à certains agents tels que la bacitracine ou le péroxyde, impliquant une augmentation de la production de vitamine K2. Les Inventeurs ont envisagé de mettre à profit cette découverte aux fins de l'obtention, en utilisant la bacitracine ou le péroxyde, par exemple, comme agent de sélection, de variants naturels de souches de bactéries lactiques (notamment *Lactococcus lactis)* capables de surproduire la vitamine K2.

### I-1- Protocole d'obtention de variants résistants à la bacitracine

Une préculture a été réalisée à partir d'un cristal d'une souche naturelle de *Lactococcus lactis* en présence de 2 mL de milieu de culture commercial classique M17 (milieu M17, Difco™) additionné de lactose à 5g/l (ci-après, milieu M17 Lac) et d'hémine (20 µL/mL) (ci-après, milieu M17 Lac + hémine). L'incubation a été effectuée sous agitation à 30°C.

La préculture a servi à ensemencer 2 mL de M17 Lac + hémine supplémenté en bacitracine (4µg/mL). Le taux d'ensemencement était de 1 %. La culture a ensuite été incubée pendant 48H sous agitation à 30°C.

Puis, 100 µL de cette suspension ont été déposés sur une gélose de M17 Lac. Un disque de papier imbibé avec 2,5 mg de bacitracine a été déposé au centre de la boîte. La gélose a été incubée 48H à 30°C. Les clones proches du disque de papier ont été cultivés en présence de bacitracine (4µg/mL) dans 2mL de M17 Lac + hémine. L'incubation a duré 24H sous agitation à 30°C.

Les cellules ont été isolées sur gélose M17 Lac en présence de bacitracine (2µg/mL) après une incubation de 48H à 30°C. Les clones isolés ont été cultivés en M17 Lac + hémine, puis incubés pendant 24H sous agitation à 30°C. Cette suspension a servi à l'élaboration du stock congelé.

Ces expériences ont permis aux Inventeurs de sélectionner le variant naturel *Lactococcus lactis subsp. cremoris* I-3557 déposé à la CNCM le 20/01/2006.

### I-2- Protocole de réalisation d'un exemple de produit laitier avec le variant « bacitracine »

Une préculture a été réalisée à partir d'un cristal de la souche dans 2 mL de M17 Lac.

La préculture a servi à ensemencer, à 1%, 50 mL de lait entier UHT qui a été incubé à 30°C pendant 24H.

Le Tableau II ci-dessous donne le résultat du dosage de vitamine K2 exprimé en µg Equivalent MK-4/100g de produit, pour le variant bacitracine-résistant et la souche correspondante sauvage.

**Tableau II**

| | | |
|---|---|---|
| **Souche** | I-3557 | sauvage |
| **Vitamine K (en µg/100g)** | 8,90 | 3,32 |

Le variant bacitracine-résistant surproduit donc, à raison d'un facteur 3, la vitamine K lorsqu'on le compare à la souche sauvage de départ.

### II- Obtention et utilisation de variants naturels résistants au peroxyde

La respiration de *Lactococcus lactis* a été mise en évidence assez récemment (Duwat *et al.,* 2001). Le séquençage du génome d'une souche de *L. lactis* (IL 1403) a confirmé la présence des gènes codant les fonctions nécessaires à la respiration aérobie (Bolotin *et al.,* 2001). *L. lactis* possède en effet les opérons *men* et *cytABCD* codant les protéines nécessaires à la synthèse de ménaquinone et à la biogenèse du cytochrome D. Cette espèce possède également les trois gènes impliqués dans les dernières étapes de la synthèse d'hème *(hemH, hemK* et *hemN,* qui sont requis dans l'oxydation de la porphyrine pour l'attachement du fer à l'hème), mais ne possède pas les gènes impliqués dans les premières étapes de ce processus. Toutefois, *L. lactis* est capable de réaliser une phosphorylation oxydative en présence de protoporphyrinogène.

On a également montré que *L. lactis* pouvait respirer en présence d'oxygène et d'hème dans le milieu de culture. Cette respiration permet aux cellules d'atteindre une biomasse plus importante et le pH final observé est plus élevé que celui habituellement obtenu. Des cultures en présence d'oxygène et/ou d'hème permettent d'obtenir des courbes de croissances comparables pendant approximativement les 6 ou 7 premières heures de fermentation. Ensuite, la consommation de glucose diminue dans le cas des cultures en présence d'oxygène et d'hème, et la production de lactate est alors moindre. Cela traduit un « shift » de métabolisme qui se produit assez tardivement au cours de la culture. La respiration de *L. lactis* se fait donc vers la fin de la phase exponentielle de croissance (Duwat *et al.,* 2001).

Le rôle de la respiration de *L. lactis* n'est pas encore connu, pas plus que le rôle que peut tenir la vitamine K2 chez cette espèce à métabolisme plutôt fermentaire. Les Inventeurs ont d'ailleurs constaté que la vitamine K2 était produite par des souches de *L. lactis* alors que la respiration n'était pas induite dans les conditions testées (pas d'hème dans le milieu et pas d'agitation permettant une bonne oxygénation du milieu).

Dans le cytoplasme, les protéines ne présentent que peu de ponts disulfures contrairement aux protéines extracellulaires. Il existe un système enzymatique largement répandu qui permet de limiter le nombre de ponts disulfures. Les liaisons S-S sont réduites en fonction SH par l'intermédiaire d'une enzyme, la thioredoxine. Cette enzyme est régénérée par la thioredoxine réductase. Vido *et al* (2005) ont créé par génie génétique un mutant *trxB1* de *L. lactis.* Le gène *trxB1* code pour la thioredoxine réductase. L'étude par électrophorèse bi-dimensionnelle des protéines synthétisées par ce mutant a montré qu'il surproduisait certaines des enzymes de la voie de synthèse de la vitamine K2, à savoir les enzymes MenB et MenD.

Au vu de ces données ainsi que d'après des observations personnelles, les Inventeurs ont supposé que l'une des voies possibles pour améliorer la production de vitamine K2 par *L. lactis* pourrait être d'induire la respiration. Une autre piste pourrait être de tenter de mobiliser la vitamine K2 pour répondre à un stress oxydatif.

Les Inventeurs ont donc cherché à obtenir des variants naturels résistants à un stress oxydatif. Il est important de noter que les variants naturels obtenus ne montraient pas de surexpression de l'opéron Men.

### II-1- Protocole d'obtention de variants résistants à un stress oxydatif

Le péroxyde a été choisi comme exemple d'agent oxydant utilisable. Bien entendu, d'autres agents oxydants tels que les ions hyperchloriques, les ions ferreux, la ménadione, le paraquat, l'oxygène ou n'importe quel autre composé oxydant approprié, pourraient être utilisés dans des conditions similaires.

Après une préculture sur milieu M17 Lac, les souches naturelles de départ ont été repiquées sur le même milieu contenant des concentrations croissantes de péroxyde (par exemple, une gamme allant de au moins 20 à au moins 25, 27, 28,5 mg/l environ). Les cultures ont été incubées à 30°C. Après 24h, les premiers tubes de la gamme de concentration ne présentant pas de croissance ont été remis à incuber pendant 24h supplémentaires. Les clones ont ensuite été isolés par épuisement sur milieu gélosé. Un clone a été sélectionné pour une concentration de péroxyde de 27 mg/l. Les Inventeurs ont noté qu'au-delà d'une concentration de péroxyde de 28,5 mg/l, il n'y avait pas de croissance.

Ces expériences ont ainsi permis aux Inventeurs de sélectionner le variant naturel *Lactococcus lactis subsp. cremoris* I-3558 déposé à la CNCM le 20/01/2006.

### II-2- Protocole de réalisation d'un exemple de produit laitier avec le variant « péroxyde »

Le clone sélectionné a été mis en croissance dans du lait entier pendant 24h. Des échantillons ont ensuite été prélevés et congelés à - 80°C pour un dosage ultérieur de vitamine K.

Le Tableau III ci-dessous indique la quantité de vitamine K2 produite par le variant résistant au péroxyde, comparée à la quantité produite par la souche de départ (quantités exprimées en µg Equivalent MK-4/100g de lait fermenté).

**Tableau III**

| **Souche** | **Vitamine K (µg/100g)** |
|---|---|
| Sauvage | 2,92 ± 0,45 |
| I-3558 | 5,94 ± 0,76 |

Comme le montre le Tableau III ci-dessus, le variant produit environ deux fois plus de vitamine K2 que la souche sauvage correspondante.

### III- Caractérisation génotypique des variants naturels I-3557 et I-3558 III-1- Matériels et méthodes

Les souches ont été cultivées une nuit à 30°C sur du milieu M17 lactose. Un lait entier du commerce a été ensemencé à l'aide de chacune de ces souches à raison de 1% de préculture. Le lait ensemencé a été placé dans des tubes de 12 mL. Les fermentations ont été arrêtées dans l'état physiologique désiré, phase exponentielle de croissance ou phase de ralentissement, en plongeant les tubes dans de l'azote liquide. Les tubes ont ensuite été stockés à -80°C jusqu'à utilisation. Des expériences précédentes avaient montré que la vitamine K est essentiellement produite en phase de ralentissement (données non montrées).

### • Extraction des ARN totaux

Tous les échantillons ont été traités de façon identique.
Ces échantillons ont été décongelés en présence de RNA protect (Qiagen - réf 76506) afin d'éviter la dégradation des ARN. Les cellules de ces échantillons ont été récupérées par centrifugation.

Ensuite, les ARN des cellules de chaque échantillon ont été isolés à l'aide du broyeur de cellules Mixer Mill MM300 (Qiagen) avec des billes (Biospec Products - ref 11079101z, Zirconia/Silica Beads diameter 0,1 mm) et en présence de Trizol^{®} (Invitrogen - ref 15596-026). La concentration et les ratios de pureté (230/260 et 260/280 nm) des ARN ont ensuite été contrôlés par spectrophotométrie avec le spectrophotomètre ND-1000 Nanodrop^{®} (Nanodrop Technologies). La qualité des ARN (RIN, ratio 16/23 S) a également été contrôlée à l'aide du 2100 Bioanalyzer - logiciel expert, version B.02.05 (Agilent Technologies) et des Kits RNA 6000 Series II Nano (Agilent Technologies - ref 5067-1511).

### • Marquages des ARNm et hybridations sur puce à ADN

Les cibles ont été synthétisées par transcription inverse des ARNm en utilisant les mêmes amorces reverse spécifiques que celles utilisées pour la synthèse des produits PCR spottés sur les puces à ADN. Ces marquages directs ont été réalisés par la société Eurogentec à l'aide du kit CyScribe first strand cDNA labelling system dCTP / purification CyScribe GFX (Amersham - ref RPN6202X) et de molécules fluorescentes couplées à des nucléotides Cy3-dCTP / Cy5-dCTP (Perkin Elmer - ref NEL576 / NEL577).

Les hybridations des cibles marquées sur les puces à ADN à produits PCR, de la souche *Lactococcus lactis cremoris* MG1363 (séquence disponible sur NCBI), ont été réalisées par la société Eurogentec, qui a elle même produit ces puces à ADN et les commercialise. Cette société a utilisé un protocole d'hybridation et de lavage classique (tampon d'hybridation Eurogentec - ref AR-HYB-01, incubation d'une nuit à 42°C en station d'hybridation Advalytix Slidebooster SB800 - Implen, lavage des puces à ADN hybridées dans un tampon 0,2X SSC / 0,1% SDS pendant 5 minutes sous agitation à température ambiante puis rinçage dans un tampon 0,2X SSC pendant 5 minutes à température ambiante sous agitation occasionnelle puis séchage des puces à ADN par centrifugation à 1 000 rpm pendant 5 minutes).

L'acquisition des données des hybridations a été réalisée par la société Eurogentec à l'aide d'un scanner Axon 4100A et du logiciel GenePix Pro 5.1 (Axon Instruments). Les scans des puces à ADN hybridées ont ensuite été envoyées aux Inventeurs par la société Eurogentec.

### • Traitement des données

Les résultats numériques issus des scans des puces à ADN hybridées ont été obtenus par les Inventeurs à l'aide du logiciel GenePix Pro 6.0.

Ces premiers résultats numériques ont suivi un traitement statistique avec le logiciel MANGO, développé par la plateforme Transcriptomique GODMAP du CNRS de Gif sur Yvette (91) de façon à obtenir des ratios d'expression significatifs ; chaque spot correspondant à un gène étant dupliqué sur les puces à ADN et chaque expérience biologique étant reproduite trois fois en incluant une inversion des marquages (DyeSwap) afin de limiter le biais d'incorporation des molécules fluorescentes couplées à des nucléotides représentant ainsi six lames par comparaison.

Les ratios d'expression différentielle ont été sélectionnés sur les critères de reproductibilité telle que la p-value ajustée (<0,01), par rapport au bruit de fond moyen et sur la valeur des ratios d'expression (r ≥ 2,00 et r ≥ 2,00).

### III-2- Résultats

Les deux variants présentent des résultats similaires quant à l'expression de certains gènes. La majorité des gènes concernés ont trait à l'état d'oxydo-réduction de la cellule et notamment en relation avec les clusters Fe-S. Dans ce cadre, le métabolisme de la méthionine et de la cystéine sont modifiés ainsi que le transport du fer. Plusieurs enzymes impliquées dans les mécanismes de défense au stress oxydant voient leur expression modifiée: thiorédoxine H, thiorédoxine B1, NADH déshydrogénase, NADH déshydrogénases, glutathione peroxydase...

En phase exponentielle, certaines enzymes impliquées dans la respiration de *L*. *Lactis* sont surexprimées : la fumarate réductase (*frdC*) et la cytochrome oxydase (*cydD*). De même, le métabolisme des bases purines semble être modifié.

Le tableau IV ci-dessous renseigne sur les ratios d'expression de certains gènes et établit une comparaison entre les variants et la souche sauvage en phase de ralentissement.

**Tableau IV**

| **Protéine** | **Gène** | **N° du gène dans la présente demande** | **N° d'accès NCBI** | **Variant I-3557** | **Variant I-3558** |
|---|---|---|---|---|---|
| O-acetylhomosérine sulfhydrylase | cysD | 1 | IImg_0091 | 4,04 | 3,70 |
| Gpo protéine (glutathione peroxydase) | gpo | 2 | IImg_1088 | 3,16 | 3,73 |
| *Gpo protein (glutathione peroxydase*) | | | | | |
| 5-methyltetrahydropteroyltriglutamate-- homocysteine méthyltransférase | metE | 3 | IImg_1225 | 2,94 | 3,12 |
| NADH déshydrogénase putative *Putative NADH dehydrogenase* | - | 4 | IImg_0195 | 2,66 | 3,77 |
| MetF protéine | | | | | |
| *MetF protein* | metF | 5 | IImg_1226 | 2,44 | 2,64 |
| *Méthylène tétrahydrofolate réductase* | | | | | |
| Thiorédoxine type H | trxH | 6 | IImg_0406 | 2,37 | 2,63 |
| *Thioredoxin H-type* | | | | | |
| Protéine de régulation de l'assimilation ferrique | fur | 7 | IImg_1023 | 2,16 | 4,91 |
| *ferric uptake regulation protein* | | | | | |
| quinone oxydoréductase | qor | 8 | IImg_1850 | 2,13 | 2,35 |
| NADH déshydrogénase | noxB | 16 | IImg_1734 | -2,01 | -2,42 |
| non-hème chlorure péroxidase | cpo | 17 | IImg_1737 | -2,39 | -2,52 |
| *non-heme chloride peroxidase* | | | | | |
| Protéine MetK | | | | | |
| *MetK protein* | metK | 18 | IImg_2160 | -3,51 | -2,07 |
| *S-adénosylméthionine synthase* | | | | | |
| Protéine TrxB1 | | | | | |
| *TrxB1 protein* | trxB1 | 19 | IImg_1588 | -3,62 | -2,94 |
| *Thiorédoxine réductase* | | | | | |
| O-acétylsérine sulfhydrylase | cysK | 20 | IImg_1775 | -3,86 | -3,54 |
| cystathionine beta-lyase | metC | 21 | IImg_1776 | -5,02 | -5,27 |
| L-lactate déshydrogénase | Idh | 27 | IImg_1120 | -10,98 | -4,31 |

Le tableau V ci-dessous donne les ratios d'expression des gènes chez les variants par comparaison avec la souche sauvage en phase exponentielle de croissance.

**Tableau V**

| **Protéine** | **Gène** | **N° du gène dans la présente demande** | **N° d'acc ès NCBI** | **Variant I-3557** | **Variant I-3558** |
|---|---|---|---|---|---|
| sous-unité flavoprotéine fumarate réductase | frdC | 9 | IImg_ 1441 | 6,28 | 6,52 |
| *fumarate reductase flavoprotein subunit* | | | | | |
| alcool-acétaldehyde | adhE | 10 | IImg_ 1916 | 4,36 | 3,14 |
| déshydrogénase | | | | | |
| *alcohol-acetaldehyde* | | | | | |
| *dehydrogenase* | | | | | |
| phosphoribosylaminoimidazole-succinocarboxamide synthase | purC | 11 | IImg_ 0973 | 3,61 | 3,13 |
| cytochrome d ubiquinol oxydase, sous-unité Il | cydB | 12 | IImg_ 1863 | 3,15 | 2,95 |
| *cytochrome d ubiquinol oxidase, subunit Il* | | | | | |
| Sous-unité catalytique | purE | 13 | IImg_ 0999 | 2,12 | 2,58 |
| phosphoribosylaminoimidazole carboxylase | | | | | |
| *phosphoribosylaminoimidazole carboxylase catalytic subunit* | | | | | |
| phosphoribosylformylglycinamidine synthase I | purQ | 14 | IImg_ 0975 | 2,29 | 2,42 |
| Thiorédoxine | trxA | 15 | IImg_ 0779 | 3,07 | 2,19 |
| *Thioredoxin* | | | | | |
| NADH déshydrogénase putative *putative NADH dehydrogenase* | - | 4 | Iimg_ 0195 | 2,41 | 2,02 |
| Protéine MetS | metS | 22 | IImg_ 1764 | -2,49 | -2,08 |
| *MetS protein* | | | | | |
| *Méthioninyl-tRNA synthétase* | | | | | |
| Homologue de la protéine B de transport de fer ferreux. | feoB | 23 | IImg_ 0199 | -3,42 | -2,21 |
| *ferrous iron transport protein B Homolog* | | | | | |
| Protéine TrxB1 | trxB1 | 19 | IImg_ 1588 | -2,74 | -2,58 |
| *TrxB1 protein* | | | | | |
| *Thiorédoxine réductase* | | | | | |
| aconitate hydratase | citB | 24 | IImg_ 0636 | -3,1 | -2,88 |
| isocitrate déshydrogénase | icd | 25 | IImg_ 0637 | -7,32 | -7,16 |
| Protéine affine du substrat du système de transport ABC du ferrichrome. | fhuD | 26 | IImg_ 0349 | -15,1 | -16,80 |
| *ferrichrome ABC transporter substrate binding protein* | | | | | |

### REFERENCES

Bolotin et al. 2001. Genome Research 11, 731-753
Duwat et al. 2001. J. Bacteriol. 183(15), 4509-16
Morishita et al. 1999. J. Dairy Sci. 82, 1897-1903
Parker et al. 2003. Journal of Food Science 68(7), 2325-2330
Vido et al. 2005. J. Bact. 187, 601-10
Gasson M. 1983. J. Bact. 154, 1-9
Hart JP, et al. [letter]. Lancet. 1984;2:283
Hart JP, et al. J Clin Endocrinol Metab. 1985;60:1268-9
Hauschka PV, et al. Physiol Rev. 1989;69:990-1047
Ducy P, et al. Nature. 1996;382:448-52
Väänänen HK, et al. Calcif Tissue Int. 1999;64:S79
Ronden JE, et al. Biochim Biophys Acta. 1998;1379:16-22
Knapen MH, et al. Ann Intern Med. 1989 Dec 15;111(12):1001-5
Szulc P, et al. J Clin Invest. 1993 Apr;91 (4):1769-74
Booth SL, et al. Am J Clin Nutr. 2000;71:1201-8
Shiraki M, et al. J Bone Miner Res. 2000;15:515-21
Braam LAJLM, et al. Calcif Tissue Int. 2003 Jul;73(1):21-6
Hirano J and IshiiY. J Orthop Sci. 2002; 7:364-369.
Tsukamoto Y, et al. Biosci. Biotechnol. Biochem. 2001; 65(9):2007-2015
Cocaign-Bousquet, M., et al. Journal of Applied Bacteriology 1995; 79, 108-116

## Revendications

1. Procédé de sélection d'un variant naturel d'une souche de bactérie lactique produisant, dans des conditions de fermentation standard, une quantité de vitamine K2 supérieure, d'un facteur au moins égal à 1,2, à celle produite par ladite souche de bactérie lactique cultivée dans les mêmes conditions, ledit procédé comprenant au moins :
a) la culture de ladite souche de bactérie lactique dans des conditions de fermentation standard, sur un milieu de sélection choisi parmi des milieux de culture contenant de la bacitracine ou un agent oxydant tel que le péroxyde, induisant une modification de l'état d'oxydo-réduction cellulaire; et
b) la sélection dudit variant s'il produit au moins 1,2 fois plus de vitamine K2 que ladite souche de bactérie lactique cultivée dans les mêmes conditions.

2. Procédé selon la revendication 1, **caractérisé en ce que** ladite souche de bactérie lactique est choisie parmi les genres *Lactococcus, Leuconostoc, Enterococcus* et *Propionibacterium.*

3. Procédé selon la revendication 2, **caractérisé en ce que** ladite souche de bactérie lactique est choisie parmi les espèces *Lactococcus lactis, Leuconostoc lactis, Leuconostoc pseudomesenteroides, Leuconostoc mesenteroides, Leuconostoc dextranicum, Enterococcus faecium, Propionibacterium sp.*

4. Procédé selon l'une quelconque des revendications 1 à 3, **caractérisé en ce que** l'expression d'au moins un gène choisi parmi les gènes n° 1 à 27 suivants :
| **N° du gène** | **Nom du gène** | **Fonction de la protéine correspondante** | **N° d'accès NCBI** |
|---|---|---|---|
| 1 | cys D | O-acétylhomosérine sulfhydrylase | IImg_0091 |
| 2 | gpo | Gpo protéine (glutathione peroxydase) | IImg_1088 |
| 3 | metE | 5-méthyltetrahydropteroyltriglutamate-homocystéine méthyltransférase | IImg_1225 |
| 4 | | NADH déshydrogénase putative | IImg_0195 |
| 5 | metF | Protéine MetF | IImg_1226 |
| | | *Méthylène tétrahydrofolate réductase* | |
| 6 | trxH | Thiorédoxine type H | IImg_0406 |
| 7 | fur | Protéine de régulation de l'assimilation ferrique | IImg_1023 |
| 8 | qor | quinone oxydoréductase | IImg_1850 |
| 9 | frdc | sous-unité flavoprotéine fumarate réductase | IImg_1441 |
| 10 | adhE | alcool-acétaldéhyde déshydrogénase | IImg_2432 |
| 11 | purC | phosphoribosylaminoimidazole-succinocarboxamide synthase | IImg_0973 |
| 12 | cydB | cytochrome d ubiquinol oxydase, sous-unité II | IImg_1863 |
| 13 | purE | Sous-unité catalytique | IImg_0999 |
| | | phosphoribosylaminoimidazole carboxylase | |
| 14 | purQ | phosphoribosylformylglycinamidine synthase I | IImg_0975 |
| 15 | trxA | Thiorédoxine | IImg_0779 |
| 16 | noxB | NADH déshydrogénase | IImg_1734 |
| 17 | cpo | non-hème chlorure péroxidase | IImg_1737 |
| 18 | metK | Protéine MetK S-adénosylméthionine synthase | IImg_2160 |
| 19 | trxB1 | Protéine TrxB1 | IImg_1588 |
| | | Thiorédoxine réductase | |
| 20 | cysK | O-acétylsérine sulfhydrylase | IImg_1775 |
| 21 | metC | cystathionine beta-lyase | IImg_1776 |
| 22 | metS | Protéine MetS | IImg_1764 |
| | | Méthionyl-tRNA synthétase | |
| 23 | feoB | Homologue de la protéine B de transport de fer ferreux. | IImg_0199 |
| 24 | citB | aconitate hydratase | IImg_0636 |
| 25 | icd | isocitrate déshydrogénase | IImg_0637 |
| 26 | fhuD | Protéine affine du substrat du système de transport ABC du ferrichrome. | IImg_0349 |
| 27 | Idh | L-lactate déshydrogénase | IImg_1120 |
est modifiée chez ledit variant par rapport à ladite souche de bactérie lactique.

5. Procédé selon la revendication 4, **caractérisé en ce que** l'expression d'au moins un gène choisi parmi les gènes n°1 à 15 est augmentée chez ledit variant par rapport à ladite souche de bactérie lactique.

6. Procédé selon la revendication 4 ou 5, **caractérisé en ce que** l'expression d'au moins un gène choisi parmi les gènes n°16 à 27 est réduite chez ledit variant par rapport à ladite souche de bactérie lactique.

7. Variant naturel d'une souche de bactérie lactique, résistant à la bacitracine, et produisant, dans des conditions de fermentation standard, une quantité de vitamine K2 supérieure, d'un facteur au moins égal à 1,2, à celle produite par ladite souche de bactérie lactique cultivée dans les mêmes conditions, **caractérisé en ce qu'**il est le variant naturel I-3557 déposé à la Collection Nationale de Culture des Microorganismes (CNCM, Institut Pasteur, 25 rue du Docteur Roux, 75724 Paris cedex 15, France) le 20/01/2006.

8. Variant naturel d'une souche de bactérie lactique, résistant à un agent oxydant tel que le péroxyde, et produisant, dans des conditions de fermentation standard, une quantité de vitamine K2 supérieure, d'un facteur au moins égal à 1,2, à celle produite par ladite souche de bactérie lactique cultivée dans les mêmes conditions, **caractérisé en ce qu'**il est le variant naturel I-3558 déposé à la Collection Nationale de Culture des Microorganismes (CNCM, Institut Pasteur, 25 rue du Docteur Roux, 75724 Paris cedex 15, France) le 20/01/2006.

9. Utilisation de la résistance à la bacitracine pour sélectionner des variants naturels de souches de bactéries lactiques produisant, dans des conditions de fermentation standard, une quantité de vitamine K2 supérieure, d'un facteur au moins égal à 1,2, à celle produite par ladite souche de bactérie lactique cultivée dans les mêmes conditions.

10. Utilisation de la résistance à un agent oxydant, tel que le péroxyde, pour sélectionner des variants naturels de souches de bactéries lactiques produisant, dans des conditions de fermentation standard, une quantité de vitamine K2 supérieure, d'un facteur au moins égal à 1,2, à celle produite par ladite souche de bactérie lactique cultivée dans les mêmes conditions.

11. Ferment lactique comprenant au moins un variant selon l'une quelconque des revendications 7 et 8.

12. Procédé de production d'un produit alimentaire enrichi en vitamine K2 comprenant au moins :
a) la mise en oeuvre d'au moins un variant selon l'une quelconque des revendications 7 et 8, et/ou au moins un ferment selon la revendication 11, dans une préparation intermédiaire dudit produit ; et
b) l'obtention dudit produit enrichi en vitamine K2.

13. Produit alimentaire enrichi en vitamine K2, comprenant au moins un variant selon l'une quelconque des revendications 7 et 8, et/ou au moins un ferment selon la revendication 11, susceptible d'être obtenu par un procédé selon la revendication 12.

## Patentansprüche

1. Verfahren zur Selektion einer natürlichen Variante eines Milchsäurebakterienstamms, der unter Standardfermentationsbedingungen eine um einen Faktor von mindestens 1,2 größere Menge Vitamin K2 produziert, als sie unter den gleichen Bedingungen von dem kultivierten Milchsäurebakterienstamm produziert wird, wobei das Verfahren mindestens Folgendes umfasst:
a) das Kultivieren des Milchsäurebakterienstamms unter Standardfermentationsbedingungen auf einem Selektionsmedium, das unter den Kulturmedien gewählt wird, die Bacitracin oder ein Oxidationsmittel, wie etwa Peroxid, enthalten und eine Modifikation des zellulären Redoxstatus bewirken; und
b) die Selektion der Variante, wenn sie mindestens 1,2-mal so viel Vitamin K2 wie der unter den gleichen Bedingungen kultivierte Milchsäurebakterienstamm produziert.

2. Verfahren nach Anspruch 1, **dadurch gekennzeichnet, dass** der Milchsäurebakterienstamm unter den Gattungen *Lactococcus, Leuconostoc, Enterococcus* und *Propionibacterium* gewählt wird.

3. Verfahren nach Anspruch 2, **dadurch gekennzeichnet, dass** der Milchsäurebakterienstamm unter den Arten *Lactococcus lactis, Leuconostoc lactis, Leuconostoc pseudomesenteroides, Leuconostoc mesenteroides, Leuconostoc dextranicum, Enterococcus faecium, Propionibacterium sp.* gewählt wird.

4. Verfahren nach einem der Ansprüche 1 bis 3, **dadurch gekennzeichnet, dass** die Expression mindestens eines Gens, das unter den folgenden Genen Nr. 1 bis 27 gewählt wird:
| **Nr. des Gens** | **Name des Gens** | **Funktion des entsprechenden Proteins** | **NCBI-Zugangsnr.** |
|---|---|---|---|
| **1** | **cys D** | **O-Acetylhomoserinsulfhydrylase** | **IImg_0091** |
| **2** | **gpo** | **Protein Gpo (Glutathionperoxidase)** | **IImg_1088** |
| **3** | **metE** | **5-Methyltetrahydropteroyltriglutamat-Homocysteinmethyltransferase** | **IImg_1225** |
| **4** | | **Putative NADH-Dehydrogenase** | **IImg_0195** |
| **5** | **metF** | **Protein MetF *Methylentetrahydrofolatreduktase*** | **IImg_1226** |
| **6** | **trxH** | **Thioredoxin Typ H** | **IImg_0406** |
| **7** | **fur** | **Eisen(III)-Assimilationsregulationsprotein** | **IImg_1023** |
| **8** | **qor** | **Chinon-Oxidoreduktase** | **IImg_1850** |
| **9** | **frdc** | **Fumeratreduktase, Flavoprotein-Untereinheit** | **IImg_1441** |
| **10** | **adhE** | **Alkohol-Acetaldehyd-Dehydrogenase** | **IImg_2432** |
| **11** | **purC** | **Phosphoribosylaminoimidazol-Succinocarboxamid-Synthase** | **IImg_0973** |
| **12** | **cydB** | **Ubichinoloxidase des Cytrochroms, Untereinheit II** | **IImg_1863** |
| **13** | **purE** | **Phosphoribosylaminoimidazol-Carboxylase, katalytische Untereinheit** | **IImg_0999** |
| **14** | **purQ** | **Phosphoribosylformylglycinamidin-Synthase I** | **IImg_0975** |
| **15** | **trxA** | **Thioredoxin** | **IImg_0779** |
| **16** | **noxB** | **NADH-Dehydrogenase** | **IImg_1734** |
| **17** | **cpo** | **Nicht-Häm-Chlorperoxidase** | **IImg_1737** |
| **18** | **metK** | **Protein MetK S-Adenosylmethionin-Synthase** | **IImg_2160** |
| **19** | **trxB1** | **Protein TrxB1 Thioredoxin-Reduktase** | **IImg_1588** |
| **20** | **cysK** | **O-Acetylserin-Sulfhydrolase** | **IImg_1775** |
| **21** | **metC** | **Cystathionin-beta-Lyase** | **IImg_1776** |
| **22** | **metS** | **Protein MetS Methionyl-tRNA-Synthetase** | **IImg_1764** |
| **23** | **feoB** | **Homolog des Eisen(II)-Transportproteins B** | **IImg_0199** |
| **24** | **citB** | **Aconitat-Hydratase** | **IImg_0636** |
| **25** | **icd** | **Isocitrat-Dehydrogenase** | **IImg_0637** |
| **26** | **fhuD** | **zum Ferrichrom-ABC-Transportsytem des Substrats affines Protein** | **IImg_0349** |
| **27** | **Idh** | **L-Laktat-Dehydrogenase** | **IImg_1120** |
bei der Variante im Vergleich zu dem Milchsäurebakterienstamm modifiziert ist.

5. Verfahren nach Anspruch 4, **dadurch gekennzeichnet, dass** die Expression mindestens eines Gens, das unter den Genen Nr. 1 bis 15 gewählt wird, bei der Variante im Vergleich zu dem Milchsäurebakterienstamm verstärkt ist.

6. Verfahren nach Anspruch 4 oder 5, **dadurch gekennzeichnet, dass** die Expression mindestens eines Gens, das unter den Genen Nr. 16 bis 27 gewählt wird, bei der Variante im Vergleich zu dem Milchsäurebakterienstamm vermindert ist.

7. Natürliche Variante eines bacitracinresistenten Milchsäurebakterienstamms, der unter Standardfermentationsbedingungen eine um einen Faktor von mindestens 1,2 größere Menge Vitamin K2 produziert, als sie unter den gleichen Bedingungen von dem kultivierten Milchsäurebakterienstamm produziert wird, **dadurch gekennzeichnet, dass** es sich um die natürliche Variante 1-3557 handelt, die bei der Collection Nationale de Culture des Microorganismes (CNCM, Institut Pasteur, 25 rue du Docteur Roux, 75724 Paris Cedex 15, Frankreich) am 20.01.2006 hinterlegt wurde.

8. Natürliche Variante eines gegen ein Oxidationsmittel, wie etwa Peroxid, resistenten Milchsäurebakterienstamms, der unter Standardfermentationsbedingungen eine um einen Faktor von mindestens 1,2 größere Menge Vitamin K2 produziert, als sie unter den gleichen Bedingungen von dem kultivierten Milchsäurebakterienstamm produziert wird, **dadurch gekennzeichnet, dass** es sich um die natürliche Variante I-3558 handelt, die bei der Collection Nationale de Culture des Microorganismes (CNCM, Institut Pasteur, 25 rue du Docteur Roux, 75724 Paris Cedex 15, Frankreich) am 20.01.2006 hinterlegt wurde.

9. Verwendung der Bacitracinresistenz zum Selektieren natürlicher Varianten von Milchsäurebakterienstämmen, die unter Standardfermentationsbedingungen eine um einen Faktor von mindestens 1,2 größere Menge Vitamin K2 produzieren, als sie unter den gleichen Bedingungen von dem kultivierten Milchsäurebakterienstamm produziert wird.

10. Verwendung der Resistenz gegen ein Oxydationsmittel, wie etwa Peroxid, zum Selektieren natürlicher Varianten von Milchsäurebakterienstämmen, die unter Standardfermentationsbedingungen eine um einen Faktor von mindestens 1,2 größere Menge Vitamin K2 produzieren, als sie unter den gleichen Bedingungen von dem kultivierten Milchsäurebakterienstamm produziert wird.

11. Milchferment, das mindestens eine Variante nach einem der Ansprüche 7 und 8 umfasst.

12. Verfahren zur Herstellung eines mit Vitamin K2 angereicherten Lebensmittels, das mindestens Folgendes umfasst:
a) Einsatz mindestens einer Variante nach einem der Ansprüche 7 und 8 und/oder mindestens eines Ferments nach Anspruch 11 in einem Zwischenerzeugnis des Lebensmittels; und
b) Erhalt des mit Vitamin K2 angereicherten Lebensmittels.

13. Mit Vitamin K2 angereichertes Lebensmittel, das mindestens eine Variante nach einem der Ansprüche 7 und 8 und/oder mindestens ein Ferment nach Anspruch 11, das durch ein Verfahren nach Anspruch 12 erhalten werden kann, umfasst.

## Claims

1. Method for obtaining a natural variant of a lactic acid bacteria producing, under standard fermentation conditions, a quantity of vitamin K2 greater, by a factor at least equal to 1.2, than that produced by said lactic acid bacteria strain cultured under the same conditions, said method comprising at least:
a) the culture of said lactic acid bacteria strain under standard fermentation conditions, on a selection medium chosen from among culture media containing bacitracin or an oxidant such as peroxide, inducing a modification of the cellular redox state; and
b) the selection of said variant if it produces at least 1.2 times more vitamin K2 than said lactic acid bacteria strain cultured under the same conditions.

2. Method according to claim 1, **characterized in that** said lactic acid bacteria strain is chosen from among the genera *Lactococcus, Leuconostoc, Enterococcus* and *Propionibacterium.*

3. Method according to claim 2, **characterized in that** said lactic acid bacteria strain is chosen from among the species *Lactococcus lactis, Leuconostoc lactis, Leuconostoc pseudomesenteroides, Leuconostoc mesenteroides, Leuconostoc dextranicum*, *Enterococcus faecium*, *Propionibacterium sp.*

4. Method according to any one of claims 1 to 3, **characterized in that** the expression of at least one gene chosen from among genes 1 to 27 below:
| Gene No. | Gene name | Function of the corresponding protein | NCBI accession No. |
|---|---|---|---|
| 1 | cys D | O-acetylhomoserine sulfhydrylase | IImg_0091 |
| 2 | gpo | Gpo protein (glutathione peroxidase) | IImg_1088 |
| 3 | metE | 5-methyltetrahydropteroyltriglutamate-homocysteine methyltransferase | IImg_1225 |
| 4 | | putative NADH dehydrogenase | IImg_0195 |
| 5 | metF | MetF Protein methylene tetrahydrofolate reductase | IImg_1226 |
| 6 | trxH | H-type thioredoxin | IImg_0406 |
| 7 | fur | Ferric uptake regulating protein | IImg_1023 |
| 8 | qor | quinone oxidoreductase | IImg_1850 |
| 9 | frdc | fumarate reductase flavoprotein subunit | IImg_1441 |
| 10 | adhE | alcohol-acetaldehyde dehydrogenase | IImg_2432 |
| 11 | purC | phosphoribosylaminoimidazole-succinocarboxamide synthase | IImg_0973 |
| 12 | cydB | cytochrome d ubiquinol oxidase, subunit II | IImg_1863 |
| 13 | purE | phosphoribosylaminoimidazole carboxylase catalytic subunit | IImg_0999 |
| 14 | purQ | phosphoribosylformylglycinamidine synthase I | IImg_0975 |
| 15 | trxA | thioredoxin | IImg_0779 |
| 16 | noxB | NADH dehydrogenase | IImg_1734 |
| 17 | cpo | non-heme peroxidase chloride | IImg_1737 |
| 18 | metK | Met K protein S-adenosylmethionine synthase | IImg_2160 |
| 19 | trxB1 | TrxB1 protein thioredoxin reductase | IImg_1588 |
| 20 | cysK | O-acetylserine sulfhydrylase | IImg_1775 |
| 21 | metC | cystathionine beta-lyase | IImg_1776 |
| 22 | metS | MetS protein methionyl-tRNA synthetase | IImg_1764 |
| 23 | feoB | Ferric iron transport protein B homolog | IImg_0199 |
| 24 | citB | aconitate hydratase | IImg_0636 |
| 25 | icd | isocitrate dehydrogenase | IImg_0637 |
| 26 | fhuD | ferrichrome ABC transporter substrate binding protein | IImg_0349 |
| 27 | Idh | L-lactate dehydrogenase | IImg_1120 |
is modified in said variant with regard to said lactic acid bacteria strain.

5. Method according to claim 4, **characterized in that** the expression of at least one gene chosen from among genes 1 to 15 is increased in said variant with regard to said lactic acid bacteria strain.

6. Method according to claim 4 or 5, **characterized in that** the expression of at least one gene chosen from among genes 16 to 27 is reduced in said variant with regard to said lactic acid bacteria strain.

7. Natural variant of a lactic acid bacteria strain, resistant to bacitracin, and producing under standard fermentation conditions, a quantity of vitamin K2 greater by a factor at least equal to 1.2 than that produced by said lactic acid bacteria strain cultured under the same conditions, **characterized in that** it is natural variant I-3557 filed with the Collection Nationale de Culture des Microorganismes (CNCM, Institut Pasteur, 25, rue du Docteur Roux, 75724 Paris Cedex 15, France) on January 20, 2006.

8. Natural variant of a lactic acid bacteria strain, resistant to an oxidant such as peroxide, and producing under standard fermentation conditions, a quantity of vitamin K2 greater by a factor at least equal to 1.2 than that produced by said lactic acid bacteria strain cultured under the same conditions, **characterized in that** it is natural variant I-3558 filed with the Collection Nationale de Culture des Microorganismes (CNCM, Institut Pasteur, 25, rue du Docteur Roux, 75724 Paris Cedex 15, France) on January 20, 2006.

9. Use of bacitracin resistance for selecting natural variants of lactic acid bacteria strains producing, under standard fermentation conditions, a quantity of vitamin K2 greater, by a factor at least equal to 1.2, than that produced by said lactic acid bacteria strain cultured under the same conditions.

10. Use of resistance to an oxidant, such as peroxide, for selecting natural variants of lactic acid bacteria strains producing, under standard fermentation conditions, a quantity of vitamin K2 greater, by a factor at least equal to 1.2, than that produced by said lactic acid bacteria strain cultured under the same conditions.

11. Lactic ferment comprising at least one variant according to any one of claims 7 and 8.

12. A method for producing a food product enriched in vitamin K2, comprising at least:
a) the use of at least one variant according to any one of claims 7 and 8, and/or at least one ferment according to claim 11, in an intermediate preparation of said product; and
b) obtaining said product enriched in vitamin K2.

13. Food product enriched in vitamin K2, comprising at least one ferment according to any one of claims 7 and 8, and/or at least one ferment according to claim 11, obtainable by a method according to claim 12.
